Europäisches Patentamt

European Patent Office

Office européen des brevets

(19)

(11) Veröffentlichungsnummer: **0 328 699 B1**

## (12) EUROPÄISCHE PATENTSCHRIFT

(45) Veröffentlichungstag der Patentschrift: **18.09.91**

(51) Int. Cl.⁵: **A61M 5/315**

(21) Anmeldenummer: **88102236.2**

(22) Anmeldetag: **16.02.88**

(54) Spritze für medizinische Zwecke.

(43) Veröffentlichungstag der Anmeldung:
**23.08.89 Patentblatt 89/34**

(45) Bekanntmachung des Hinweises auf die
Patenterteilung:
**18.09.91 Patentblatt 91/38**

(84) Benannte Vertragsstaaten:
**AT BE CH DE ES FR GB GR IT LI LU NL SE**

(56) Entgegenhaltungen:
EP-A- 0 115 931
FR-A- 2 178 019
US-A- 2 752 920
US-A- 4 226 236

(73) Patentinhaber: **Arzneimittel GmbH Apotheker
Vetter & Co. Ravensburg
Marienplatz 79-81
W-7980 Ravensburg(DE)**

(72) Erfinder: **Vetter, Udo J.
Schützenstrasse 99-101
7980 Ravensburg(DE)**

(74) Vertreter: **Fay, Hermann, Dipl.-Phys. Dr. et al
Dipl.-Phys. Hermann Fay und Dr. Joachim
Dziewior Ensingerstrasse 21 Postfach 17 67
W-7900 Ulm/Donau(DE)**

## Beschreibung

Die Erfindung betrifft eine Spritze für medizinische Zwecke, mit einem am einen Ende zum Ansatz einer Injektionsnadel ausgebildeten Spritzenzylinder und mit einem im Spritzenzylinder verschiebbaren Spritzenkolben, der axial fest an einer Kolbenstange sitzt, die an dem dem Nadelansatzende entgegengesetzten Zylinderende aus dem Spritzenzylinder hervorsteht, sowie mit einem zweiten Spritzenkolben, der zwischen dem Nadelansatzende und dem an der Kolbenstange sitzenden Spritzenkolben ebenfalls verschiebbar angebracht ist und so eine Zweikammerspritze bildet, wobei in der Wand des Spritzenzylinders eine einen Bypass bildende, radial nach außen gerichtete Auswölbung vorgesehen ist, über die beide Kammern bei im Bereich der Auswölbung stehendem zweiten Spritzenkolben miteinander verbunden sind.

Eine Spritze dieser Art ist aus der EP-A-0 191 122 bekannt und insbesondere dazu vorgesehen, als Einmalspritze in sterilisiertem und mit der Injektionssubstanz vorgefülltem gebrauchsfertigem Zustand hergestellt, aufbewahrt und angewendet zu werden. Derartige Zweikammerspritzen finden insbesondere dann Anwendung, wenn die Injektionssubstanz aus einer Mischung in flüssiger Phase oder einer Lösung besteht, die nur kurze Zeit haltbar ist und daher erst kurz vor der Injektion hergestellt werden darf. In diesem Fall ist jede der beiden Substanzkomponenten in jeweils einer eigenen Kammer untergebracht, wobei diese Kammern im Spritzenzylinder axial hintereinander angeordnet sind und erst unmittelbar vor der Injektion so miteinander in Verbindung gebracht werden, daß die flüssige Substanz aus der hinteren Kammer in die die andere, vorzugsweise trockene Substanz enthaltende nadelseitig vordere Kammer überströmen und die gewünschte Mischung oder Lösung in dieser vorderen Kammer bilden kann. Hierzu dient der im Spritzenzylinder vorgesehene Bypass, über den das Überströmen der flüssigen Substanz dann möglich ist, wenn der die beiden Kammern trennende Spritzenkolben so weit zum Nadelansatzende hin verschoben ist, daß er in axialer Richtung von beiden Enden des Bypasses übergriffen wird. Nachteilig bei diesen bekannten Spritzen ist jedoch, daß bei zu starkem Druck auf die Kolbenstange die flüssige Substanz über den Bypass zu schnell in die nadelseitige vordere Kammer überströmen und dadurch schon gelöste Substanz der vorderen Kammer unkontrolliert durch die Kanüle ins Freie treten kann. Dadurch ist eine unerwünschte Kontamination sowohl des Anwenders als auch des Patienten möglich.

Der Erfindung liegt die Aufgabe zugrunde, eine Spritze der eingangs genannten Art so auszubilden, daß die Überströmgeschwindigkeit der flüssigen Substanz in die vordere, die trockene Substanz enthaltende Kammer begrenzt wird, ohne daß dies jedoch einen Einfluß darauf hat, mit welcher Geschwindigkeit die fertig gelöste Substanz anschließend injiziert wird.

Diese Aufgabe wird nach der Erfindung dadurch gelöst, daß an dem dem Nadelansatzende entgegengesetzten Zylinderende ein als Kolbenbremse dienendes Dämpfungsteil angeordnet ist, das bei einer Verstellung des Spritzenkolbens mittels der Kolbenstange in Richtung zum Nadelansatzende hin eine zunächst nur verringerte Hubgeschwindigkeit der Kolbenstange zuläßt, so lange der an der Kolbenstange sitzende Spritzenkolben den Bereich des Bypasses noch nicht erreicht hat.

Der durch die Erfindung erreichte Fortschritt besteht im wesentlichen darin, daß durch die zunächst begrenzte Hubgeschwindigkeit des bzw. der Spritzenkolben die Flüssigkeit so langsam von der hinteren in die vordere Kammer überströmt, daß die Gefahr eines vorzeitigen unkontrollierten Austritts schon gelöster Injektionssubstanz aus der Kanüle ausgeschlossen oder doch zumindest erheblich verringert ist. Weiter ergibt sich hierbei der Vorteil, daß der trockenen Substanz mehr Zeit zur Verfügung steht, um in Lösung zu gehen, so daß der Wirkstoff sich in der flüssigen Substanz homogener verteilen kann.

In bevorzugter Ausführungsform der Erfindung ist das Dämpfungsteil von einer Verschlußkappe gebildet, die auf das dem Nadelansatzende entgegengesetzte Zylinderende aufgesetzt ist, dabei zugleich mit ihrem radial über den Spritzenzylinder vorstehenden Rand eine Fingerauflage bildet und mit einem Innengewinde für einen an der Kolbenstange an dessen an den Spritzenkolben angrenzenden Bereich angeordneten Gewindeschaft versehen ist, wobei die Länge des Gewindeschafts so bemessen ist, daß sein dem Spritzenkolben abgewandtes Ende aus dem Innengewinde frei kommt, wenn dieser in axialer Richtung etwa den Bypass erreicht hat. Um die flüssige Substanz aus der hinteren Kammer in die nadelseitig vordere Kammer zu überführen, muß daher die Kolbenstange um ihre Achse gedreht werden, wodurch die Hubgeschwindigkeit durch die Drehgeschwindigkeit einerseits sowie die Ganghöhe des Gewindes andererseits bestimmt ist. Sobald der Gewindeschaft aus dem Gewinde der Verschlußkappe freigekommen ist, kann anschließend die Injektion in üblicher Weise erfolgen.

Hierfür kann es sich weiter empfehlen, daß der Durchmesser der Kolbenstange in dem an den Gewindeschaft anschließenden Bereich nur geringfügig kleiner als der lichte Durchmesser des Innengewindes ist. Hierdurch erfährt die Kolbenstange eine die Handhabung der Spritze fördernde Führung.

Ferner ist es im Rahmen der Erfindung von Vorteil, wenn der Spritzenkolben mit einem axial angeordneten Sackloch für einen von der Kolbenstange vorstehenden Gewindebolzen versehen ist, wobei das Sackloch mit einem an seinem Boden freigeschnittenen Innengewinde versehen ist und der Gewindebolzen an seinem an der Kolbenstange angeschlossenen Ende ebenfalls eine Freischneidung für das Innengewinde des Sacklochs aufweist. Auf diese Weise kann die Kolbenstange einfach in den Spritzenkolben eingeschraubt werden und ist anschließend durch die beiderseitigen Freischneidungen frei darin drehbar, jedoch in axialer Richtung fest mit diesem verbunden.

Schließlich kann in einer vorteilhaften Weiterbildung der Erfindung die Verschlußkappe mit einem axial ins Innere des Spritzenzylinders vorstehenden, die Kolbenstange umschließenden Kragen versehen sein, der auf seiner Innenseite einen Teil des Innengewindes trägt und der mit der Wand des Spritzenzylinders eine Ringnut bildet. Durch diese Ringnut lassen sich verbleibende Reste der Injektionssubstanz aus dem Bypass auffangen, die je nach Haltung der Spritze in deren hinteren Bereich abfließen können. Somit wird auch an dieser Stelle der Spritze eine Kontamination des Anwenders durch austretende Injektionslösung verhindert.

Im folgenden wird die Erfindung an einem in der Zeichnung dargestellten Ausführungsbeispiel näher erläutert; es zeigen:

Fig. 1    eine Spritze nach der Erfindung in vorgefülltem Zustand,

Fig. 2    den Gegenstand nach Fig. 1, nachdem der Inhalt der ersten Kammer in die zweite Kammer überführt ist,

Fig. 3    den Gegenstand nach Fig. 1 in injektionsbereiter Stellung.

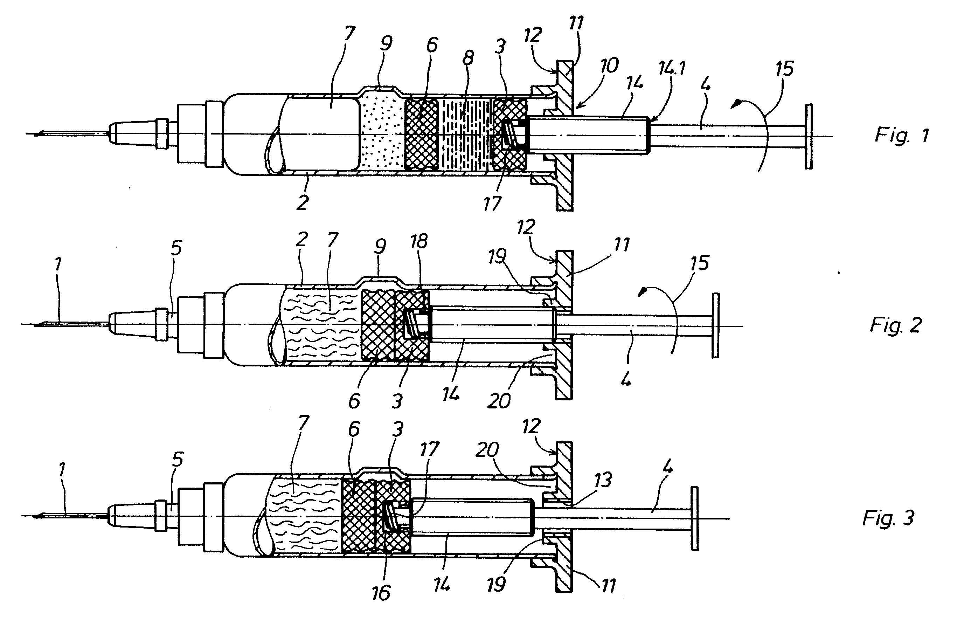

Die in der Zeichnung dargestellte Spritze ist für medizinische Zwecke vorgesehen und dazu mit einem am einen Ende zum Ansatz einer Injektionsnadel 1 ausgebildeten Spritzenzylinder 2 und mit einem im Spritzenzylinder 2 verschiebbaren Spritzenkolben 3 versehen. Der Spritzenkolben 3 sitzt axial fest an einer Kolbenstange 4, die an dem dem Nadelansatzende 5 entgegengesetzten Zylinderende aus dem Spritzenzylinder 2 hervorsteht. Ferner ist ein zweiter Spritzenkolben 6 vergesehen, der zwischen dem Nadelansatzende 5 und dem an der Kolbenstange 4 sitzenden Spritzenkolben 3 ebenfalls verschiebbar angeordnet ist und so eine Zweikammerspritze bildet. Solche Zweikammerspritzen finden insbesondere dann Anwendung, wenn die Injektionssubstanz aus einer Mischung in flüssiger Phase oder einer Lösung besteht, die nur kurze Zeit haltbar ist und daher erst kurz vor der Injektion hergestellt werden darf. Dabei finden solche Zweikammerspritzen insbesondere als Einmalspritzen Anwendung, die in sterilisiertem und mit der Injektionssubstanz vorgefülltem, gebrauchsfertigem Zustand hergestellt, aufbewahrt, und angewendet werden. Die beiden Kammern 7, 8 liegen im Spritzenzylinder 2 axial hintereinander und werden erst unmittelbar vor der Injektion über einen Bypass 9 miteinander in Verbindung gebracht, so daß die flüssige Substanz aus der hinteren Kammer 8 in die die andere, vorzugsweise trockene Substanz enthaltende nadelseitig vordere Kammer 7 überströmen und dort die gewünschte Mischung oder Lösung bilden kann. Der Bypass 9 ist dabei von einer in der Wand des Spritzenzylinders 2 radial nach außen gerichteten Auswölbung gebildet. Um die beiden Kammern 7, 8 über den Bypass 9 miteinander in Verbindung zu bringen, muß der zweite Spritzenkolben 6 so weit zum Nadelansatzende 5 hin verschoben werden, daß die beiden Enden des Bypasses 9 zu beiden Seiten des Spritzenzylinders hin münden, wie dies aus Fig. 2 ersichtlich ist.

An dem dem Nadelansatzende 5 entgegengesetzten Zylinderende ist ein als Kolbenbremse dienendes Dämpfungsteil 10 vorgesehen, das bei einer Verstellung des Spritzenkolbens 3 aus der in Fig. 1 dargestellten Stellung mittels der Kolbenstange 4 in Richtung zum Nadelansatzende 5 hin eine zunächst nur verringerte Hubgeschwindigkeit der Kolbenstange 4 zuläßt, so lange der an der Kolbenstange 4 sitzende Spritzenkolben 3 den Bereich des Bypasses 9 noch nicht erreicht hat.

Im einzelnen ist das Dämpfungsteil 10 von einer Verschlußkappe 11 gebildet, die auf das dem Nadelansatzende 5 entgegengesetzte Zylinderende aufgesetzt ist. Die Verschlußkappe 11 bildet dabei zugleich mit ihrem radial über den Spritzenzylinder 2 vorstehenden Rand eine bei Injektionsspritzen übliche Fingerauflage 12. Ferner ist die Verschlußkappe 11 mit einem Innengewinde 13 für einen an der Kolbenstange 4 in dessen an den Spritzenkolben 3 angrenzenden Bereich angeordneten Gewindeschaft 14 versehen. Die Länge des Gewindeschaftes 14 ist dabei so bemessen, daß sein dem Spritzenkolben 3 abgewandtes Ende 14.1 aus dem Innengewinde 13 freikommt, wenn dieser in axialer Richtung etwa den Bypass 9 erreicht hat. Um daher den Spritzenkolben 3 aus der in Fig. 1 dargestellten Stellung in diejenige der Fig. 2 zu verstellen, muß die Kolbenstange 4 zunächst um ihre Längsachse gedreht werden, wie dies durch den Pfeil 15 angedeutet ist. Hierdurch wird erreicht, daß die flüssige Substanz aus der hinteren Kammer 8 ausreichend langsam in die vordere Kammer 7 überströmt, so daß keine schon gelöste Injektionssubstanz unkontrolliert durch die Kanüle 1 ins Freie treten kann. Sobald der Gewindeschaft 14 aus dem Innengewinde 13 ausgetreten ist, wie dies in Fig. 3 dargestellt ist, kann mit der Spritze in üblicher Weise die Injektion verabreicht werden.

Der Durchmesser der Kolbenstange 4 kann in seinem an den Gewindeschaft 14 anschließenden Bereich nur geringfügig kleiner als der lichte Durchmesser des Innengewindes 13 gewählt werden, wodurch sich für den Gewindeschaft 14 eine zusätzliche, die Handhabung der Spritze bei der Injektion fördernde Führung ergibt. Der Spritzenkolben 3 ist mit einem axial angeordneten Sackloch 16 für einen von der Kolbenstange 4 vorstehenden Gewindebolzen 17 versehen, wobei das Sackloch 16 mit einem an seinem Boden freigeschnittenen Innengewinde 18 versehen ist. Entsprechend weist der Gewindebolzen 17 an seinem an der Kolbenstange 4 angeschlossenen Ende ebenfalls eine Freischneidung für das Innengewinde 18 des Sackloches 16 auf. Dadurch ist die freie Drehbarkeit der in den Spritzenkolben 3 eingeschraubten Kolbenstange 4 gegeben, wie sie in der Anfangsphase zum Überführen der flüssigen Substanz in die vordere Kammer 7 erforderlich ist. Zugleich sind der Spritzenkolben 3 und die Kolbenstange 4 aber in axialer Richtung fest miteinander verbunden.

Die Verschlußkappe 11 ist mit einem axial ins Innere des Spritzenzylinders 2 vorstehenden, die Kolbenstange 4 umschließenden Kragen 19 versehen, der auf seiner Innenseite einen Teil des Innengewindes 13 trägt und der mit der Wand des Spritzenzylinders 2 eine Ringnut 20 bildet. In diese Ringnut 20 können aus dem Bypass 9 stammende Reste der Injektionssubstanz aufgefangen werden, die sonst unter Umständen durch die mit dem Innengewinde 13 versehene Öffnung der Verschlußkappe 11 nach außen gelangen und zu einer unerwünschten Kontamination mit dem Anwender führen könnten.

**Patentansprüche**

1. Spritze für medizinische Zwecke, mit einem am einen Ende zum Ansatz einer Injektionsnadel (1) ausgebildeten Spritzenzylinder (2) und mit einem im Spritzenzylinder (2) verschiebbaren Spritzenkolben (3), der axial fest an einer Kolbenstange (4) sitzt, die an dem dem Nadelansatzende (5) entgegengesetzten Zylinderende aus dem Spritzenzylinder (2) hervorsteht, sowie mit einem zweiten Spritzenkolben (6), der zwischen dem Nadelansatzende (5) und dem an der Kolbenstange (4) sitzenden Spritzenkolben (3) ebenfalls verschiebbar angebracht ist und so eine Zweikammerspritze bildet, wobei in der Wand des Spritzenzylinders (2) eine einen Bypass (9) bildende, radial nach außen gerichtete Auswölbung vorgesehen ist, über die beide Kammern (7, 8) bei im Bereich der Auswölbung stehendem zweiten Spritzenkolben (6) miteinander verbunden sind, dadurch gekennzeichnet, daß an dem dem Nadelansatzende (5) entgegengesetzten Zylinderende ein als Kolbenbremse dienendes Dämpfungsteil (10) angeordnet ist, das bei einer Verstellung des Spritzenkolbens (3) mittels der Kolbenstange (4) in Richtung zum Nadelansatzende (5) eine zunächst nur verringerte Hubgeschwindigkeit der Kolbenstange (4) zuläßt, solange der an der Kolbenstange (4) sitzende Spritzenkolben (3) den Bereich des Bypasses (9) nicht erreicht hat.

2. Spritze nach Anspruch 1, dadurch gekennzeichnet, daß das Dämpfungsteil (10) von einer Verschlußkappe (11) gebildet ist, die auf das dem Nadelansatzende (5) entgegengesetzte Zylinderende aufgesetzt ist, dabei zugleich mit ihrem radial über den Spritzenzylinder (2) vorstehenden Rand eine Fingerauflage (12) bildet und mit einem Innengewinde (13) für einen an der Kolbenstange (4) in dessen an den Spritzenkolben (3) angrenzenden Bereich angeordneten Gewindeschaft (14) versehen ist, wobei die Länge des Gewindeschafts (14) so bemessen ist, daß sein dem Spritzenkolben (3) abgewandtes Ende aus dem Innengewinde (13) freikommt, wenn dieser in axialer Richtung etwa den Bypass (9) erreicht hat.

3. Spritze nach Anspruch 2, dadurch gekennzeichnet, daß der Durchmesser der Kolbenstange (4) in dem an den Gewindeschaft (14) anschließenden Bereich nur geringfügig kleiner als der lichte Durchmesser des Innengewindes (13) ist.

4. Spritze nach Anspruch 2 oder 3, dadurch gekennzeichnet, daß der Spritzenkolben (3) mit einem axial angeordneten Sackloch (16) für einen von der Kolbenstange (4) vorstehenden Gewindebolzen (17) versehen ist, wobei das Sackloch (16) mit einem an seinem Boden freigeschnittenen Innengewinde (18) versehen ist und der Gewindebolzen (17) ein seinem an der Kolbenstange (4) angeschlossenen Ende ebenfalls eine Freischneidung für das Innengewinde (18) des Sacklochs (16) aufweist.

5. Spritze nach den Ansprüchen 2 bis 4, dadurch gekennzeichnet, daß die Verschlußkappe (11) mit einem axial ins Innere des Spritzenzylinders (2) vorstehenden, die Kolbenstange (4) umschließenden Kragen (19) versehen ist, der auf seiner Innenseite einen Teil des Innengewindes (13) trägt und der mit der Wand des Spritzenzylinders (2) eine Ringnut (20) bildet.

**Claims**

1. A syringe for medical purposes comprising a syringe cylinder (2) which is designed at one end for the attachment of an injection needle (1), and a syringe plunger (3) which is displaceable in the syringe cylinder (2) and which is axially fixedly carried on a plunger rod (4) which projects out of the syringe cylinder (2) at the end thereof which is opposite to the needle attachment end (5), and further comprising a second syringe plunger (6) which is also displaceably disposed between the needle attachment end (5) and the Syringe plunger (3) carried on the plunger rod (4) and thus forms a two-chamber syringe, wherein provided in the wall of the syringe cylinder (2) is a radially outwardly directed bulge portion which forms a by-pass (9) and by way of which both chambers (7, 8) are connected together when the second syringe plunger (6) is in the region of the bulge portion, characterised in that arranged at the end of the cylinder which is opposite to the needle attachment end (5) is a damping portion (10) which serves as a plunger brake and which, upon displacement of the syringe plunger (3) by means of the plunger rod (4) in a direction towards the needle attachment end (5) permits an initially only reduced stroke speed of the plunger rod (4) as long as the syringe plunger (3) carried on the plunger rod (4) has not reached the region of the by-pass (9).

2. A syringe according to claim 1 characterised in that the damping portion (10) is formed by a closure cap (11) which is fitted on to the end of the cylinder which is opposite to the needle attachment end (5), at the same time forms a finger support (12) with its edge projecting radially beyond the syringe cylinder (2), and is provided with an internal screwthread (13) for a screwthreaded shank (14) arranged on the plunger rod (4) in the region thereof adjoining the syringe plunger (3), wherein the length of the screwthreaded shank (14) is such that its end remote from the syringe plunger (3) comes free from the internal screwthread (13) when the latter has approximately reached the by-pass (9) in the axial direction.

3. A syringe according to claim 2 characterised in that the diameter of the plunger rod (4), in the region adjoining the screwthreaded shank (14), is only slightly smaller than the inside diameter of the internal screwthread (13).

4. A syringe according to claim 2 or claim 3 characterised in that the syringe plunger (3) is provided with an axially arranged blind hole (16) for a screwthreaded pin (17) projecting from the plunger rod (4), wherein the blind hole (16) is provided with an internal screwthread (18) which is cut free at its bottom, and the screwthreaded pin (17) is also provided at its end connected to the plunger rod (4) with a portion which is cut free, for the internal screwthread (18) of the blind hole (16).

5. A syringe according to claim 2 to 4 characterised in that the closure cap (11) is provided with a flange (19) which projects axially into the interior of the syringe cylinder (2) and which embraces the plunger rod (4) and which on its inside carries a part of the internal screwthread (13) and which forms an annular groove (20) with the wall of the syringe cylinder (2).

**Revendications**

1. Seringue à usage médical, comportant, à une extrémité, un cylindre (2) agencé pour le raccordement d'une aiguille d'injection (1), et un piston (3) pouvant être déplacé dans le cylindre (2) et qui est monté, d'une manière fixe axialement, sur une tige de piston (4) qui fait saillie hors du cylindre (2) à l'extrémité de ce cylindre tournée à l'opposé de l'extrémité (5) de fixation de l'aiguille, ainsi qu'un second piston (6), qui est également monté de manière à être déplaçable sur le cylindre (3) monté sur la tige de piston (4) et forme ainsi une seringue à deux chambres, dans laquelle il est prévu, dans la paroi du cylindre (2), une partie cintrée qui délimite une dérivation (9) et est dirigée radialement vers l'extérieur et au moyen de laquelle deux chambres (7,8) sont reliées entre elles lorsque le second piston (6) est situé dans la zone de la partie renflée, caractérisée en ce que sur l'extrémité du cylindre, tournée à l'opposé de l'extrémité (5) de fixation de l'aiguille, se trouve disposé un élément d'amortissement (10), qui est utilisé en tant que frein pour le piston et qui, dans le cas d'un déplacement du piston (3) à l'aide de la tige de piston (4) en direction de l'extrémité (5) de fixation de l'aiguille, autorise le déplacement de la tige de piston (4) tout d'abord seulement avec une vitesse d'avance réduite, tant que le piston (3), qui prend appui sur la tige de piston (4), n'a pas atteint la zone de la dérivation (9).

2. Seringue selon la revendication 1, caractérisée en ce que l'élément d'amortissement (10) est formé par un capuchon de fermeture (11), qui est installé sur l'extrémité du cylindre tournée à l'opposé de l'extrémité (5) de fixation de l'aiguille, et forme simultanément, par son bord qui fait saillie radialement par rapport au cylindre (2) de la seringue, un appui (12) pour un doigt et comporte un taraudage (13) pour une tige filetée (14) disposée sur la tige de piston (4) dans sa partie jouxtant le piston (3), la longueur de la tige filetée (14) étant dimensionnée de sorte que son extrémité tournée à l'opposé du piston (3) sort du taraudage (13) lorsque ce piston a atteint approximativement la dérivation (9), dans la direction axiale.

3. Seringue selon la revendication 2, caractérisée en ce que le diamètre de la tige de piston (4) dans la zone se raccordant à la tige filetée (14) est seulement légèrement inférieur au diamètre supérieur du taraudage (13).

4. Seringue selon la revendication 2 ou 3, caractérisée en ce que le piston (3) est équipé d'un trou borgne axial (16) servant à loger un boulon fileté (17) qui fait saillie à partir de la tige de piston (4), le trou borgne (16) comportant un taraudage (18) interrompu à proximité de son front, tandis que le boulon fileté (17) possède, au niveau de son extrémité raccordée à la tige de piston (4), également une partie dégagée pour le taraudage (18) du trou borgne (16).

5. Seringue selon les revendications 2 à 4, caractérisée en ce que le capuchon de fermeture (11) comporte un collet (19), qui pénètre axialement à l'intérieur du cylindre (2), entoure la tige de piston (4), comporte, sur son côté intérieur, une partie du taraudage (13) et délimite, avec la paroi du cylindre (2), une gorge annulaire (20).

Fig. 1

Fig. 2

Fig. 3